# EUROPÄISCHE PATENTSCHRIFT

(11) **EP 2 664 453 B1**
(45) Veröffentlichungstag und Bekanntmachung des Hinweises auf die Patenterteilung: **12.02.2020**
(21) Anmeldenummer: 13001894.8
(22) Anmeldetag: 11.04.2013
(51) Int. Cl.: B32B 7/12, B32B 25/08, B32B 27/28, C08L 65/04, B32B 27/08

(54) **FLEXIBLES LAMINAT UND VERFAHREN ZU SEINER HERSTELLUNG**
FLEXIBLE LAMINATE AND METHOD OF MANUFACTURING SAME
LAMINÉ FLEXIBLE ET SON PROCÉDÉ DE FABRICATION

(30) Priorität: 15.05.2012 DE 102012010825
(43) Veröffentlichungstag der Anmeldung: 20.11.2013
(73) Patentinhaber: Ottobock SE & Co. KGaA, 37115 Duderstadt (DE)
(72) Erfinder: Dornieden, Dionys, 37339 Neuendorf (DE)
(74) Vertreter: Lins, Edgar

(56) Entgegenhaltungen:
- EP-A1- 1 806 224
- WO-A1-2007/025059
- WO-A1-2012/005614
- WO-A1-2012/140262
- US-A1- 2005 043 581
- US-A1- 2010 174 239
- US-B2- 6 586 048
- DATABASE WPI Week 200837 Thomson Scientific, London, GB; AN 2008-F67059 & JP 2008 064423 A (OSAKA GAS CO LTD) 21 March 2008 (2008-03-21)

## Beschreibung

Die Erfindung betrifft ein flexibles Laminat, gebildet aus wenigstens einer ersten Lage eines ersten flexiblen Elastomers und einer zweiten Lage aus einem zweiten flexiblen Elastomer, die mit einer auf die erste Lage aufgebrachten Zwischenschicht miteinander verbunden sind.

Die Erfindung betrifft ferner ein Verfahren zur Herstellung eines flexiblen Laminats aus wenigstens einer ersten Lage eines ersten flexiblen Elastomers und einer zweiten Lage aus einem zweiten flexiblen Elastomer, die mit einer auf die erste Lage aufgebrachten Zwischenschicht miteinander verbunden sind.

Es ist bekannt, dass bestimmte elastische Kunststoffmaterialien sich nicht unproblematisch miteinander verbinden lassen. So ist es beispielsweise nicht möglich, eine Silikonschicht und eine Polyurethanschicht direkt oder mit Klebstoffen miteinander zu verbinden. Die Beschichtung von Silikon ist daher immer problematisch.

Ein ähnliches Problem besteht für die Beschichtung von thermoplastischen Elastomeren, wie sie beispielsweise auf der Basis von Styrol-Isopren-Butadien-Blockcopolymeren oder Styrol-Ethylen/Butylen-Copolymer gebildet sind. Diese thermoplastischen Elastomere (TPE) können einen hohen Ölgehalt aufweisen, durch den eine flächige Verbindung mit anderen Materialien problematisch wird.

Durch US 2009/0240344 A1 ist eine Verbindung der Materialien mit einer textilen Zwischenschicht bekannt, die mit beiden Materialien verbindbar ist. Dadurch verliert allerdings das gebildete Laminat an Flexibilität. Darüber hinaus wird die Schichtdicke vergrößert. Aus derselben Schrift ist es ferner bekannt, eine der Schichten mit einer texturierten Oberfläche, beispielsweise in Form von zahlreichen Haken, zu versehen, um dadurch eine mechanische Verankerung der danach aufgegossenen Schicht aus dem anderen Kunststoffmaterial zu erzielen. Ein derartiges Verfahren ist aufwendig.

Durch DE 26 17 678 A1 ist es bekannt, ein Polymer-Verbundmaterial aus zwei polymeren Komponenten herzustellen, von denen eine ein vulkanisierter Kautschuk und die andere ein unterschiedlicher vulkanisierter Kautschuk oder ein Kunststoff ist. Die Verbindung der polymeren Komponenten wird dadurch hergestellt, dass eine teilchenförmige Bindeschicht an der Grenzfläche der Komponenten vorgesehen wird, die vorwiegend Teilchen von Kohlenstoff und/oder einer Siliziumverbindung enthält. Diese Schicht wird von beiden Komponenten durchdrungen.

Durch DE 10 2008 063 818 A1 ist es bekannt, ein orthopädisches Polster aus einem fluidgefüllten Elastomer, einem Elastomergel, einem weichgemachten Thermoplasten oder einem Polyurethan mit einer reibvermindernden Schicht zu versehen, indem das auspolymerisierte Polster wenigstens einem Vorhandlungsschritt unterworfen wird, in dem flüchtige Bestandteile aus der Oberfläche entzogen werden und das Polster anschließend mit einer unpolaren, die Gleitfähigkeit erhobenen Polymerbeschichtung beschichtet wird. Das polymere Beschichtungsmaterial kann dabei Polyparaxylylen (gebräuchlicher Trivialname: Parylen) sein.

Durch WO 2012/140262 A1 ist es bekannt, in ein Elastomer eine Verstärkunqsfolie, die aus Polyethylen, Polypropylen. Parylen C bestehen kann, einzubringen, indem zunächst eine erste Lage des Elastomers gegossen wird, dann die mit Durchgangslöchern versehene Verstärkungsfolie aufgelegt wird und anschließend eine weitere Lage desselben Elastomers aufgebracht wird, sodass sich eine einheitliche Elastomerlage bildet, die durch die Durchgangslöcher der Verstärkungsfolie Verbindungsstege bildet, sodass eine mechanische formschlüssige Fixierung der Verstärkungsfolie innerhalb des Elastomers entsteht. Eine materialschlüssige Verbindung zwischen der Verstärkungsfolie und dem Elastomer ist nicht offenbart. Ferner besteht wegen der identischen Elastomerlage auch kein Verbindungsproblem zwischen verschiedenen Elastomeren.

Der vorliegenden Erfindung liegt die Aufgabe zugrunde, eine flächige Verbindung eines ersten flexiblen Elastomers mit einem zweiten flexiblen Elastomer zu einem flexiblen Laminat zu ermöglichen, bei der die Flexibilität durch die Zwischenschicht nicht merkbar beeinträchtigt und durch die Zwischenschicht keine wesentliche Vergrößerung der Dicke des Laminats erforderlich ist.

Zur Lösung dieser Aufgabe ist das flexible Laminat der eingangs erwähnten Art dadurch gekennzeichnet, die Zwischenschicht eine Parylenschicht mit einer durch eine beim Aufbringen der Parylenschicht gebildete Oberflächenrauheit verursachten unregelmäßigen Oberfläche ist und dass das zweite Elastomer oder ein mit der zweiten Lage verbundener Kleber die Unregelmäßigkeiten der Oberfläche der Parylenschicht zumindest teilweise so ausfüllt, dass das zweite Elastomer oder der mit dem zweiten Elastomer verbundene Kleber mit der Oberfläche der Parylenschicht verhakt und so eine vorwiegend mechanische Verbindung der ersten Lage mit der zweiten Lage mittels einer mechanischen Verbindung zu der Parylenschicht bewirkt.

Die Aufgabe wird ferner mit einem Verfahren der eingangs erwähnten Art dadurch gelöst, dass auf die erste Lage eine Parylenschicht so aufgetragen wird, dass die Parylenschicht eine durch eine unregelmäßige Oberfläche verursachte Oberflächenrauheit aufweist und dass das zweite Elastomer oder ein mit der zweiten Lage verbundener Kleber in flüssiger Form auf die Parylenschicht so aufgebracht wird, dass die Unregelmäßigkeiten der Oberfläche der Parylenschicht zumindest teilweise so ausgefüllt werden, dass das zweite Elastomer oder der mit dem zweiten Elastomer verbundene Kleber mit der Oberfläche der Parylenschicht verhakt und so eine vorwiegend mechanische Verbindung der ersten Lage mit der zweiten Lage mittels einer mechanischen Verbindung zu der Parylenschicht bewirkt.

Erfindungsgemäß wird somit eine Parylenschicht verwendet, die als Haftvermittler fungiert. Dies ist überraschend, weil Parylen als reibungsverminderndes Material bekannt ist, das somit auf ihrer Oberfläche eine nur geringe Haftung zulässt.

Der vorliegenden Erfindung liegt jedoch die Erkenntnis zugrunde, dass die Parylenschicht, insbesondere wenn sie im CVD-Verfahren (Chemical Vapor Deposition) aufgebracht wird, eine unregelmäßige Oberfläche ausbilden kann. Die so gebildeten Oberflächen stehen einer Verminderung des Reibungskoeffizienten durch die Parylenfläche nicht entgegen, ermöglichen jedoch einem flüssigen Polymer das Ausfüllen der Unregelmäßigkeiten, sodass sich das zweite Elastomer in flüssiger Form oder ein mit der zweiten Lage des zweiten Elastomers verbundener Kleber in flüssiger Form mit der Oberfläche der Parylenschicht verhaken kann und so eine vorwiegend mechanische Verbindung der zweiten Lage mit der ersten Lage bewirkt. Die Unregelmäßigkeiten können Hinterschneidungen aufweisen, die das Verhaken der Materialien miteinander unterstützen. Die Hinterschneidungen können dabei auch durch Poren gebildet sein, die sich von der Oberfläche aus zum Materialinnern hin vergrößern. Beim Aufbringen der Parylenschicht im CVD-Verfahren entsteht darüber hinaus eine Oberfläche mit einer unregelmäßigen, gitterähnlichen Struktur aus sich kreuzenden Furchen, die ebenfalls die mechanische Verbindung der Lagen miteinander bewirken oder zumindest unterstützen.

Es hat sich herausgestellt, dass sich schwierig zu beschichtende erste Elastomere stabil mit Parylen beschichten lassen, insbesondere wenn die Parylenschicht durch CVD auf die erste Lage aufgebracht wird. Somit lassen sich insbesondere Silikone und thermoplastische Elastomere mit einer Parylenschicht direkt beschichten, wobei die Parylenschicht aufgrund ihrer Unregelmäßigkeiten zumindest auch eine mechanische Verbindung zu dem zweiten Elastomer oder dem mit ihm verbundenen Kleber herstellt, wenn das zweite Elastomer oder der Kleber in die Unregelmäßigkeiten der Parylenschicht eingedrungen ist.

Es ist daher erforderlich, dass das zweite Elastomer oder der die Verbindung zum zweiten Elastomer herstellende Kleber in flüssiger Form auf das mit der Parylenschicht beschichtete erste Elastomer aufgebracht wird, sodass das Eindringen in die Unregelmäßigkeiten ermöglich wird und das zweite Elastomer oder der Kleber die Unregelmäßigkeiten zumindest teilweise ausfüllt. Eine verhakende Verbindung kommt dabei insbesondere dann zustande, wenn die Unregelmäßigkeiten Hinterschneidungen, beispielsweise in Form von porenartigen Zwischenräumen, aufweisen.

Das erste Elastomer ist vorzugsweise ein Silikon oder ein thermoplastisches Elastomer. Dieses erste Elastomer wird mit der Parylenschicht, vorzugsweise durch CVD, beschichtet. Dadurch ist das erste Elastomer mit einem zweiten Elastomer verbindbar.

Als zweites Elastomer kommt insbesondere Polyurethan oder ein thermoplastisches Elastomer in Frage, wofür die Verwendung eines thermoplastischen Elastomers als zweiten Elastomer insbesondere Silikon als erstes Elastomer bevorzugt ist. Es kann, insbesondere für ein thermoplastisches Elastomer als zweites Elastomer, sinnvoll sein, dass zweite Elastomer ebenfalls mit einer Parylenschicht, vorzugsweise durch CVD, zu beschichten und dann das beschichtete erste Elastomer und das beschichtete zweite Elastomer mit einem geeigneten Kleber, insbesondere Polyurethankleber, zu verbinden.

Die auf die erste Lage aufgebrachte Parylenschicht hat vorzugsweise eine Dicke zwischen 0,4 und 5 µm, weiter bevorzugt zwischen 0,7 und 4 µm, insbesondere 0,7 und 2 µm. Dabei kann die Parylenschicht in ihrer dünnen Version in einem einmaligen Auftrag mittels CVD aufgetragen werden, während für größere Schichtdicken ein zweimaliges oder dreimaliges Beschichten sinnvoll ist. Für die geringe Schichtdicke von 0,7 µm wird in einem Ausführungsbeispiel ein Rz-Wert von 11,4 und ein Wert für die mittlere Rauheit Ra von 1,49 erzielt.

In dem bevorzugten Schichtdickenbereich wird die Elastizität der beiden Lagen aus Elastomeren nicht durch die Zwischenschicht merklich verringert. Dies gilt auch, wenn für die Verbindung mit dem zweiten Elastomer mit dem durch die Parylenschicht beschichteten ersten Elastomers ein Kleber verwendet wird, der vorzugsweise auf Polyurethan-Basis hergestellt ist.

Als Parylen (Polyparaxylylen) kommt insbesondere das Parylen N als erfindungsgemäßer Haftvermittler in Frage. Versuche haben ergeben, dass auch die Varianten Parylen F und Parylen C nahezu gleichwertig verwendbar sind. Das erfindungsgemäße flexible Laminat kann als Flachmaterial oder als vorgeformtes Material Verwendung finden. Die Verbindung der beiden Elastomere miteinander zu dem flexiblen Laminat bietet den Vorteil, dass unterschiedliche Vorteile der beiden Elastomere in einem Laminat verwendbar sind. So kann die gute Anpassbarkeit einer Polyurethanschicht mit den guten Rückstelleigenschaften und der relativen Unempfindlichkeit einer Silikonschicht kombiniert werden. In ähnlicher Weise kann die gute Hautverträglichkeit eines thermoplastischen Elastomers, das einen hohen Weißölgehalt aufweist mit der mechanischen Stabilität und Reißfestigkeit der Silikonschicht verbunden werden. Bevorzugte Kombinationen sind daher Silikon als erstes Elastomer mit Polyurethan als zweiten Elastomer und TPE als erstes Elastomer mit Silikon als zweitem Elastomer.

Eine besonders bevorzugte Verwendung des erfindungsgemäßen flexiblen Laminats ist die Verwendung als Liner für eine Prothese, die ein amputiertes Gliedmaß ersetzt. Der Anschluss der Prothese erfolgt dabei über einen Prothesenschaft, der einen Amputationsstumpf, beispielsweise an einem Unterschenkel oder an einem Oberschenkel bei einer Beinprothese umfasst. Der Liner wird auf den Amputationsstumpf aufgebracht, beispielsweise wegen seiner Elastizität aufgerollt, um so fest am Amputationsstumpf anzuliegen und den Unregelmäßigkeiten des Amputationsstumpfs anzupassen. Der Liner befindet sich somit beispielweise unmittelbar auf der Haut des Amputationsstumpfs und sollte daher eine gute hautverträgliche Innenlage aufweisen. Diese kann beispielsweise aus Polyurethan oder aus TPE bestehen. Weist der Liner, wie dies mit dem erfindungsgemäßen flexiblen Laminat möglich ist, eine Außenlage beispielsweise aus Silikon auf, bietet das Silikon eine mechanisch stabile Außenlage des Liners, die darüber hinaus gute Rückstellfähigkeiten hat, um so die polsternde Innenlage aus Polyurethan oder TPE an den Amputationsstumpf anzudrücken, wodurch sich die Innenlage an die Unregelmäßigkeiten der Oberfläche des Amputationsstumpfs anpasst.

In manchen Fällen kann es auch sinnvoll sein, die polsternden Eigenschaften des Polyurethans oder des TPE mit den Rückstelleigenschaften des Silikons dadurch auszunutzen, dass das hautfreundliche und leicht zu reinigende Silikon die Innenlage des Liners bildet und die polsternde Elastomerlage aus Polyurethan oder TPE die Außenlage bildet. In diesem Fall kann es im Übrigen sinnvoll sein, die Außenlage mit einer Gleitschicht zu versehen, beispielsweise durch eine Textilschicht oder eine Parylenbeschichtung, die dann nicht erfindungsgemäß als Haftvermittler sondern - wie im Stand der Technik bekannt - als Gleitschicht fungiert. Durch die Gleitschicht kann beispielsweise das Einführen des mit dem Liner versehenen Amputationsstumpfs in den Prothesenschaft oder das Entfernen des Amputationsstumpfs aus dem Prothesenschaft erleichtert werden.

Für diese Anwendung sind thermoplastische Elastomere bevorzugt, die generell SEBS (Styrol-Ethylen/Butylen-Styrol) oder SIBS (Styrol-Isopren/Butadien-Styrol) basierte thermoplastische Elastomere sind, insbesondere Styrol-Isopren-Butadien-Blockcopolymere und Styrol-Ethylen/Butylencopolymer. Derartige in Frage kommende TPEs sind beispielsweise Evopren-TPE-Zusammensetzungen der AlphaGery Ltd., England, Polymere der Serie SEBS G der KRATON Polymers GmbH, Deutschland und Septon 4044 der Kuraray Europe GmbH, Deutschland.

In der Zeichnung ist ein Ausführungsbeispiel des erfindungsgemäßen flexiblen Laminats in Form eines Liners dargestellt. Es zeigen
- Figur 1: einen Längsschnitt durch einen Liner aus einem erfindungsgemäßen flexiblen Laminat;
- Figur 2: einen vergrößerten Längsschnitt-Ausschnitt für eine Variante des Liners gemäß Figur 1;
- Figur 3: einen Längsschnitt durch einen Liner gemäß Figur 2.

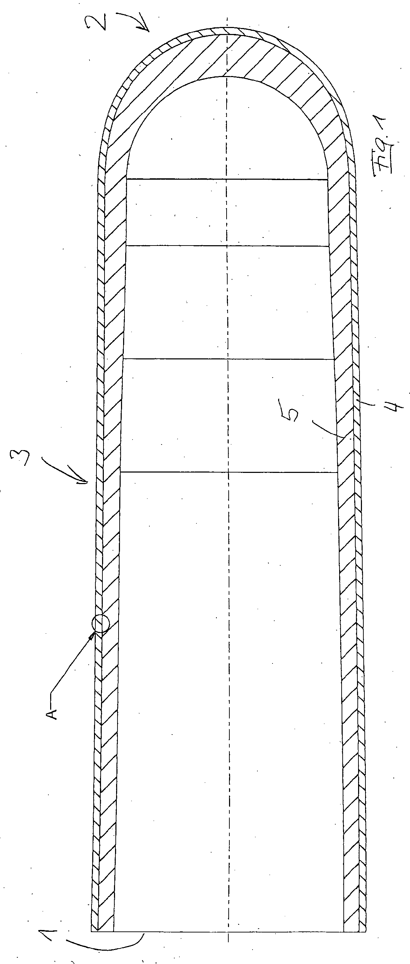

Der in Figur 1 dargestellte Liner weist ein proximales offenes Ende 1, ein distales geschlossenes Ende 2 und ein zwischen den Enden 1,2 im Wesentlichen zylindrisch oder etwas konisch verlaufendes Mittelstück 3 auf.

Der Liner besteht aus einer ersten, äußeren Lage 4 aus einem ersten Elastomer, beispielsweise Silikon und aus einer zweiten, inneren Lage 5 aus einem zweiten, hautfreundlichen Elastomer, beispielsweise Polyurethan. Es ist erkennbar, dass die zweite Lage 5 zum distalen Ende 2 hin an Dicke zunimmt, um die Polsterung des Amputationsstumpfes zum distalen Ende hin zu verbessern und einen höheren Komfort zu gewährleisten.

Die erste Lage 4 und die zweite Lage 5 können über die gesamte Länge des Liners flächig miteinander verbunden sein.

Die flächige Verbindung kann dabei die aneinanderliegenden Flächen der ersten Lage und der zweiten Lage vollständig oder nur teilweise erfassen. So kann es ausreichend sein, die erste Lage 4 und die zweite Lage 5 nur teilweise miteinander zu verbinden, beispielsweise in Form von Streifen oder über die Fläche verteilte Verbindungspunkte. Diese Variante ist insbesondere bei der Verwendung eines Klebers naheliegend.

Die flächige Verbindung wird dadurch ermöglicht, dass die erste Lage 4 mit einer (nicht näher dargestellten) eine unregelmäßige Oberflächen aufweisenden Parylenschicht, insbesondere im CVD-Verfahren als Zwischenschicht beschichtet worden ist. Wenn die Parylenschicht im CVD-Verfahren aufgebracht worden ist, weist sie eine sinterähnliche Struktur mit porenartigen, sich zum Materialinneren erweiternden Zwischenräumen auf, die als Hinterschneidungen wirken. Die zweite Lage 5, beispielsweise aus Polyurethan, kann dann auf die beschichtete erste Lage unmittelbar, d.h. ohne Kleber, aufgebracht werden, wenn die zweite Lage dabei in einer Form erst gebildet wird, sodass das zweite Elastomer der zweiten Lage 5 in flüssiger Form mit der durch die Parylenschicht gebildeten Innenseite der ersten Lage 4 in Kontakt gebracht wird. Dadurch kann das flüssige Material des zweiten Elastomers der zweiten Lage 5 teilweise in die Unregelmäßigkeit der aufgebrachten Parylenschicht eindringen und so für eine feste Verbindung zwischen der zweiten Lage 5 und der ersten Lage 4 sorgen.

Es ist allerdings auch möglich, die zweite Lage 5 in einer komplementären Form zur ersten Lage 4 vorzuproduzieren und im geformten Zustand in die erste Lage 4 einzukleben. Dabei wird ein Kleber verwendet, der einerseits mit dem zweiten Elastomer der zweiten Lage 5 eine feste Verbindung hervorbringt und andererseits im flüssigen Zustand in die Poren der Parylenschicht auf der Innenseite der ersten Lage 4 eindringt.

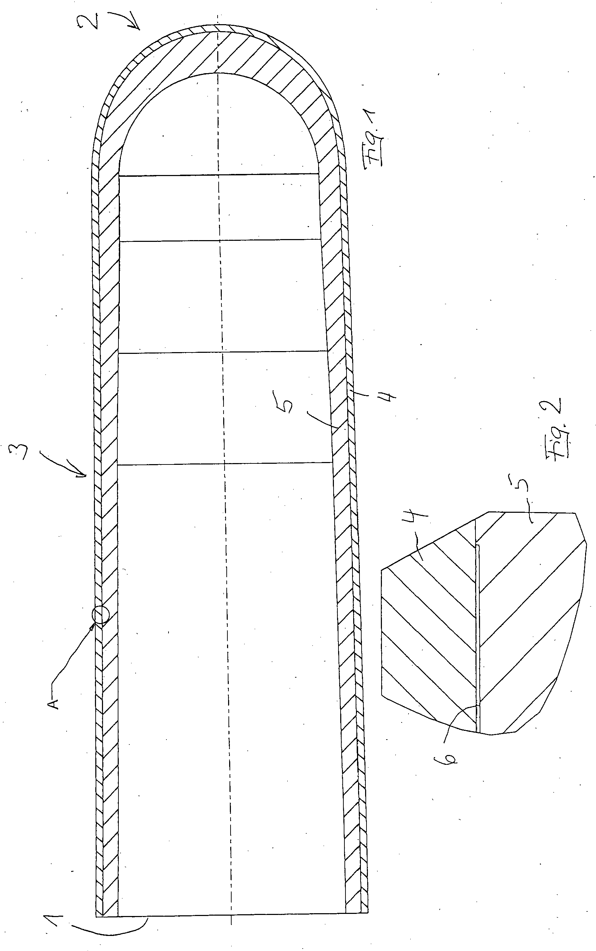

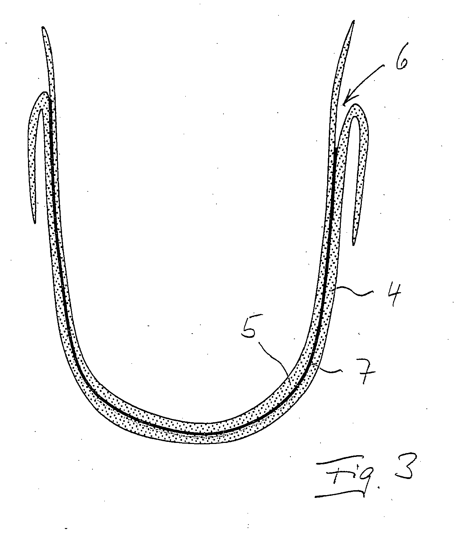

Insbesondere bei der beschriebenen Verklebung der zweiten Lage 5 mit der ersten Lage 4 kann die in den Figuren 2 und 3 dargestellte Variante verwirklicht werden, bei der die Verklebung flächig über das distale Ende 2 und den größten Teil des Mantelbereichs 3 erfolgt, jedoch zum proximalen Ende hin im Bereich A in Figur 1 beendet wird, sodass zum proximalen Ende hin die erste Lage 4 als äußere Lage und die zweite Lage 5 als innere Lage nicht miteinander verbunden sind, wie dies durch den Zwischenraum 6 zwischen der äußeren ersten Lage 4 und der inneren zweiten Lage 5 in der vergrößerten Darstellung der Figur 2 erkennbar ist. Wie Figur 3 verdeutlicht, ist es möglich, die äußere erste Lage 4 in dem Bereich, in dem die beiden Lagen 4, 5 nicht flächig miteinander verbunden sind, also im Bereich des Zwischenraums 6 nach außen umzuklappen, um beispielsweise eine Abdichtung des proximalen Randes des (nicht dargestellten) Prothesenschafts zu bewirken, wenn zwischen dem Liner und dem Prothesenschaft ein Unterdruck erzeugt werden soll, durch den der Prothesenschaft sicher an dem mit dem Liner versehen Amputationsstumpf im Gebrauch gehalten wird.

Figur 3 verdeutlicht, dass die Verbindung zwischen den Lagen 4 und 5 mittels eines Klebers 7 erfolgen kann.

Es hat sich herausgestellt, dass durch die erfindungsgemäße Parylen-Zwischenschicht auch sonst nicht miteinander verbindbare Elastomere fest miteinander verbindbar sind und Scherkräften widerstehen, die zwischen 0,2 und 1,4 N/mm liegen. Diese Verbindungskraft ist für die Verwendung als Liner bei weitem ausreichend, die jedoch ohne die erfindungsgemäße Parylen-Zwischenschicht bei der Verbindung von Materialpaarungen wie Silikon-Polyurethan, TPE-Silikon und TPE-Polyurethan nicht erreichbar ist, weil bei diesen Materialpaarungen keine merkbare Haftwirkung erreicht wird.

## Patentansprüche

1. Flexibles Laminat gebildet aus wenigstens einer ersten Lage (4) eines ersten flexiblen Elastomers und einer zweiten Lage (5) aus einem zweiten flexiblen Elastomer, die mit einer auf die erste Lage (4) aufgebrachten Zwischenschicht miteinander verbunden sind, **dadurch gekennzeichnet, dass** die Zwischenschicht eine Parylenschicht mit einer durch eine beim Aufbringen der Parylenschicht gebildete Oberflächenrauheit verursachten unregelmäßigen Oberfläche ist und dass das zweite Elastomer oder ein mit der zweiten Lage (5) verbundener Kleber die Unregelmäßigkeiten der Oberfläche der Parylenschicht zumindest teilweise so ausfüllt, dass das zweite Elastomer oder der mit dem zweiten Elastomer verbundene Kleber mit der Oberfläche der Parylenschicht verhakt und so eine vorwiegend mechanische Verbindung der ersten Lage (4) mit der zweiten Lage (5) mittels einer mechanischen Verbindung zu der Parylenschicht bewirkt.

2. Flexibles Laminat nach Anspruch 1, **dadurch gekennzeichnet, dass** die Parylenschicht durch CVD auf die erste Lage (4) aufgebracht ist.

3. Flexibles Laminat nach Anspruch 1 oder 2, **dadurch gekennzeichnet, dass** die erste Lage (4) aus Silikon oder einem thermoplastischen Elastomer (TPE) besteht.

4. Flexibles Laminat nach einem der Ansprüche 1 bis 3, **dadurch gekennzeichnet, dass** die zweite Lage (5) aus Polyurethan oder TPE besteht.

5. Flexibles Laminat nach einem der Ansprüche 1 bis 4, **dadurch gekennzeichnet, dass** es als Liner für eine Prothese ausgebildet ist.

6. Flexibles Laminat nach Anspruch 5, **dadurch gekennzeichnet, dass** die zweite Lage (5) eine Innenschicht bildet, die durch die erste Lage (4) außen abgedeckt ist.

7. Verfahren zur Herstellung eines flexiblen Laminats aus wenigstens einer ersten Lage (4) eines ersten flexiblen Elastomers und einer zweiten Lage (5) aus einem zweiten flexiblen Elastomer, die mit einer Zwischenschicht miteinander verbunden sind, **dadurch gekennzeichnet, dass** auf die erste Lage (4) eine Parylenschicht so aufgetragen wird, dass die Parylenschicht eine durch eine unregelmäßige Oberfläche verursachte Oberflächenrauheit aufweist und dass das zweite Elastomer oder ein mit der zweiten Lage (5) verbundener Kleber in flüssiger Form auf die Parylenschicht so aufgebracht wird, dass die Unregelmäßigkeiten der Oberfläche der Parylenschicht zumindest teilweise so ausgefüllt werden, dass das zweite Elastomer oder der mit dem zweiten Elastomer verbundene Kleber mit der Oberfläche der Parylenschicht verhakt und so eine vorwiegend mechanische Verbindung der ersten Lage (4) mit der zweiten Lage (5) mittels einer mechanischen Verbindung zu der Parylenschicht bewirkt.

8. Verfahren nach Anspruch 7, **gekennzeichnet durch** die Verwendung eines Silikons oder eines TPE für die erste Lage (4).

9. Verfahren nach Anspruch 7 oder 8, **gekennzeichnet durch** die Verwendung eines TPE oder eines Polyurethan für die zweite Lage (5).

10. Verfahren zur Herstellung eines flexiblen Laminats nach einem der Ansprüche 7 bis 9 in Form eines Liners für eine Prothese.

## Claims

1. Flexible laminate, formed by at least a first layer (4) of a first flexible elastomer and a second layer (5) of a second flexible elastomer, which are connected to one another by an intermediate layer applied to the first layer (4), **characterized in that** the intermediate layer is a parylene layer with an irregular surface formed during the application of the parylene layer and **in that** the second elastomer or an adhesive connected to the second layer (5) at least partially fills the irregularities of the surface of the parylene layer, so that the second elastomer or the adhesive connected to the second elastomer gets caught by the surface of the parylene layer thereby effecting a predominantly mechanical connection of the first layer (4) with the second layer (5) by means of the mechanical connection with the parylene layer.

2. Flexible laminate according to claim 1, **characterized in that** the parylene layer is applied to the first layer (4) by CVD.

3. Flexible laminate according to claim 1 or 2, **characterized in that** the first layer (4) consists of silicone or a thermoplastic elastomer (TPE).

4. Flexible laminate according to one of claims 1 to 3, **characterized in that** the second layer (5) consists of polyurethane or TPE.

5. Flexible laminate according to one of claims 1 to 4, **characterized in that** it is formed as a liner for a prosthesis.

6. Flexible laminate according to claim 5, **characterized in that** the second layer (5) forms an inner layer, which is covered on the outside by the first layer (4) .

7. Method for producing a flexible laminate from at least a first layer (4) of a first flexible elastomer and a second layer (5) of a second flexible elastomer, which are connected to one another by an intermediate layer, **characterized in that** a parylene layer is applied to the first layer (4) such that the parylene layer has a surface roughness caused by an irregular surface and **in that** the second elastomer or an adhesive connected to the second layer (5) is applied in liquid form to the parylene layer such that the irregularities of the surface of the parylene layer are at least partially filled, so that the second elastomer or the adhesive connected to the second elastomer gets caught by the surface of the parylene layer thereby effecting a predominantly mechanical connection of the first layer (4) with the second layer (5) by means of the mechanical connection with the parylene layer.

8. Method according to claim 7, **characterized by** the use of a silicone or a PTE for the first layer (4).

9. Method according to claim 7 or 8, **characterized by** the use of a TPE or a polyurethane for the second layer (5).

10. Method for producing a flexible laminate according to one of claims 7 to 9 in the form of a liner for a prosthesis.

## Revendications

1. Produit stratifié flexible formé d'au moins une première couche (4) d'un premier élastomère flexible et d'une seconde couche (5) d'un second élastomère flexible, qui sont reliées l'une à l'autre par une couche intermédiaire appliquée sur la première couche (4),
**caractérisé en ce que**
la couche intermédiaire est une couche de parylène présentant une surface irrégulière engendrée par une rugosité de surface qui se forme lors de l'application de la couche de parylène, et **en ce que** le second élastomère ou une colle reliée à la seconde couche (5) remplit au moins partiellement les irrégularités de la surface de la couche de parylène de telle façon que le second élastomère ou la colle reliée au second élastomère établit une relation d'accrochage avec la surface de la couche de parylène et entraîne ainsi une liaison principalement mécanique de la première couche (4) avec la seconde couche (5) au moyen d'une liaison mécanique vis-à-vis de la couche de parylène.

2. Produit stratifié flexible selon la revendication 1, **caractérisé en ce que** la couche de parylène est appliquée par CVD (déposition chimique en phase vapeur) sur la première couche (4).

3. Produit stratifié flexible selon la revendication 1 ou 2, **caractérisé en ce que** la première couche (4) est constituée de silicone ou d'un élastomère thermoplastique (TPE).

4. Produit stratifié flexible selon l'une des revendications 1 à 3, **caractérisé en ce que** la seconde couche (5) est constituée de polyuréthane ou de TPE.

5. Produit stratifié flexible selon l'une des revendications 1 à 4, **caractérisé en ce qu'**il est réalisé à titre de doublage pour une prothèse.

6. Produit stratifié flexible selon la revendication 5, **caractérisé en ce que** la seconde couche (5) forme une couche intérieure qui est recouverte à l'extérieur par la première couche (4).

7. Procédé pour la fabrication d'un produit stratifié flexible à partir d'au moins une première couche (4) d'un premier élastomère flexible et d'une seconde couche (5) d'un second élastomère flexible, qui sont reliées l'une à l'autre par une couche intermédiaire,
**caractérisé en ce qu'**une couche de parylène est appliquée sur la première couche (4) de telle façon que la couche de parylène présente une rugosité de surface provoquée par une surface irrégulière, et **en ce que** le second élastomère ou une colle reliée à la seconde couche (5) est appliqué(e) sous forme liquide sur la couche de parylène de telle façon que les irrégularités de la surface de la couche de parylène sont remplies au moins partiellement, de telle manière que le second élastomère ou la colle reliée au second élastomère établit une relation d'accrochage avec la surface de la couche de parylène et provoque ainsi une liaison essentiellement mécanique de la première couche (4) avec la seconde couche (5) au moyen d'une liaison mécanique.

8. Procédé selon la revendication 7, **caractérisé par** l'utilisation d'un silicone ou d'un TPE pour la première couche (4).

9. Procédé selon la revendication 7 ou 8, **caractérisé par** l'utilisation d'un TPE ou d'un polyuréthane pour la seconde couche (5).

10. Procédé pour la fabrication d'un produit stratifié flexible selon l'une des revendications 7 9 sous la forme d'un doublage pour une prothèse.
